# EUROPÄISCHE PATENTSCHRIFT

(11) **EP 1 096 010 B1**
(45) Veröffentlichungstag und Bekanntmachung des Hinweises auf die Patenterteilung: **15.09.2010**
(21) Anmeldenummer: 00122057.3
(22) Anmeldetag: 11.10.2000
(51) Int. Cl.: C12N 15/31, C07K 14/34, C12P 13/06, C12P 13/08, C12N 15/10

(54) **Für den Export verzweigtkettiger Aminosäuren kodierende Nukleotidsequenzen, Verfahren zu deren Isolierung und ihre Verwendung**
Nucleotide sequences coding for branched-chain amino acids export proteins, method for isolating them and their use
Séquences nucléotides codantes pour des protéines d'exportation d'acides aminés à chaînes ramifiées, procédé permettant d'isoler ces séquences et leur utilisation

(30) Priorität: 27.10.1999 DE 19951708
(43) Veröffentlichungstag der Anmeldung: 02.05.2001
(73) Patentinhaber: Evonik Degussa GmbH, 45128 Essen (DE); Forschungszentrum Jülich GmbH, 52425 Jülich (DE)
(72) Erfinder: Kennerknecht, Nicole, 47798 Krefeld (DE); Eggeling, Lothar, Dr., 52428 Jülich (DE); Sahm, Hermann, Prof., 52428 Jülich (DE); Pfefferle, Walter, Dr., 33790 Halle (Westf.) (DE)

(56) Entgegenhaltungen:
- EP-A- 1 090 993
- EP-A- 1 108 790
- DE-A- 19 548 222
- DATABASE EMBL [Online] accession: O07942, 15. Juli 1998 (1998-07-15) BELITSKY B R ET AL: "BRANCHED-CHAIN AMINO ACID TRANSPORT PROTEIN AZLC." XP002159588
- DATABASE EMBL [Online] accession: O07923, 15. Juli 1998 (1998-07-15) BELITSKY B R ET AL: "BRANCHED-CHAIN AMINO ACID TRANSPORT PROTEIN AZLD." XP002159589
- TAUCH ANDREAS ET AL: "Isoleucine uptake in Corynebacterium glutamicum ATCC 13032 is directed by the brnQ gene product." ARCHIVES OF MICROBIOLOGY, Bd. 169, Nr. 4, April 1998 (1998-04), Seiten 303-312, XP000979580 ISSN: 0302-8933
- EGGELING L ET AL: "Transport mutants and transport genes of Corynebacterium glutamicum." ANNALS OF THE NEW YORK ACADEMY OF SCIENCES, Bd. 782, 1996, Seiten 191-201, XP000981900 Conference;Deauville, France; October 16-21, 1994, biotechnology, III. The integration of biological and engineering sciences. 1996 New York Academy of Sciences 2 East 63rd Street, New York, New York 10021, USA ISBN: 0-89766-962-2
- EGGELING L. ET AL: "The fruits of molecular physiology: engineering the L-isoleucine biosynthesis pathway in Corynebacterium glutamicum" JOURNAL OF BIOTECHNOLOGY, vol. 56, no. 3, 28 August 1997 (1997-08-28), pages 167-182, XP004103426 ISSN: 0168-1656
- HERMANN T. ET AL: "Mechanism and Regulation of isoleucine excretion in Corynebacterium glutamicum" APPLIED AND ENVIRONMENTAL MICROBIOLOGY, vol. 62, no. 9, September 1996 (1996-09), pages 3238-3244, XP000891666 ISSN: 0099-2240

### SEQUENZPROTOKOLL

<110> Degussa-Hüls AG Forschungszentrum-Jülich GmbH
<120> Für den Export verzweigtkettiger Aminosäuren kodierende Nukleotidsequenzen, Verfahren zu deren Isolierung und ihre Verwendung
<130> 990128 BT
<140>
   <141>
<160> 6
<170> PatentIn Ver. 2.1
<210> 1
   <211> 1271
   <212> DNA
   <213> Corynebacterium glutamicum ATCC14752
<220>
   <221> gene
   <222> (101)..(853)
   <223> brnF
<220>
   <221> gene
   <222> (853)..(1176)
   <223> brnE
<400> 1
   gcgcgatcaa tggaatctag cttcatatat tgcacaatag cctagttgag gtgcgcaaac 60
   tggcaacaaa actacccggc aattgtgtga tgattgtagt gtgcaaaaaa cgcaagagat 120
   tcattcaagc ctggaggtgt cgccatccaa ggcagccctg gaaccagatg ataaaggtta 180
   tcggcgctac gaaatcgcgc aaggtctaaa aacctccctt gctgcaggtt tgggcatgta 240
   cccgattggt attgcgtttg gtctcttggt tattcaatac ggctacgaat ggtgggcagc 300
   cccactgttt tccggcctga ttttcgcggg ctccaccgaa atgctggtca tcgccctcgt 360
   tgtgggcgca gcgcccctgg gcgccatcgc gctcaccaca ttgctggtga acttccgcca 420
   cgtattctat gcgttttcat tcccgctgca tgtggtcaaa aaccccattg cccgtttcta 480
   ttcggttttc gcgcttatcg acgaagccta cgcagtcact gcggccaggc ccgcaggctg 540
   gtcggcgtgg cgacttatct caatgcaaat agcgtttcac tcctactggg tattcggcgg 600
   tctcaccgga gtggcgatcg cagagttgat tccttttgaa attaagggcc tcgagttcgc 660
   cctttgctct ctctttgtca cgctgacttt ggattcctgc cgaacgaaaa agcagatccc 720
   ttctctgctg ctcgcaggtt tgagcttcac cattgctctt gtggtaattc caggtcaggc 780
   cctatttgcg gcgctgctga tcttcttggg tctgttgacc atccggtact tcttcttggg 840
   aaaggctgct aaatgacaac tgatttctcc tgtattctcc ttgttgtcgc agtatgtgca 900
   gtcattactt ttgcgctccg ggcggttccg ttcttaatcc ttaagcccct acgtgaatca 960
   caatttgtgg gcaaaatggc gatgtggatg ccagcaggaa tccttgccat tttgaccgca 1020
   tcaacgtttc gcagcaatgc gatagatctg aagactctaa cctttggtct cattgccgtt 1080
   gcgattacag tggtggcgca tcttcttggc ggtcgacgca ccttgttgag cgttggcgct 1140
   ggcaccatcg tttttgttgg actggtgaat cttttctaaa actgcataaa taacaaaaat 1200
   ccgcatgccc tcaatttgaa ggggatgcgg attttttaag gaacctagaa aaggcttaag 1260
   cagacagcgc t                                                      1271
<210> 2
   <211> 753
   <212> DNA
   <213> Corynebacterium glutamicum ATCC14752
<220>
   <221> CDS
   <222> (1)..(753)
   <223> brnF
<400> 2
   gtg caa aaa acg caa gag att cat tca agc ctg gag gtg tcg cca tcc      48
   Val Gln Lys Thr Gln Glu Ile His Ser Ser Leu Glu Val Ser Pro Ser
     1               5                  10                  15
   aag gca gcc ctg gaa cca gat gat aaa ggt tat cgg cgc tac gaa atc      96
   Lys Ala Ala Leu Glu Pro Asp Asp Lys Gly Tyr Arg Arg Tyr Glu Ile
                20                  25                  30
   gcg caa ggt cta aaa acc tcc ctt gct gca ggt ttg ggc atg tac ccg     144
   Ala Gln Gly Leu Lys Thr Ser Leu Ala Ala Gly Leu Gly Met Tyr Pro
            35                  40                  45
   att ggt att gcg ttt ggt ctc ttg gtt att caa tac ggc tac gaa tgg     192
   Ile Gly Ile Ala Phe Gly Leu Leu Val Ile Gln Tyr Gly Tyr Glu Trp
        50                  55                  60
   tgg gca gcc cca ctg ttt tcc ggc ctg att ttc gcg ggc tcc acc gaa     240
   Trp Ala Ala Pro Leu Phe Ser Gly Leu Ile Phe Ala Gly Ser Thr Glu
    65                  70                  75                  80
   atg ctg gtc atc gcc ctc gtt gtg ggc gca gcg ccc ctg ggc gcc atc     288
   Met Leu Val Ile Ala Leu Val Val Gly Ala Ala Pro Leu Gly Ala Ile
                    85                  90                  95
   gcg ctc acc aca ttg ctg gtg aac ttc cgc cac gta ttc tat gcg ttt     336
   Ala Leu Thr Thr Leu Leu Val Asn Phe Arg His Val Phe Tyr Ala Phe
               100                 105                 110
   tca ttc ccg ctg cat gtg gtc aaa aac ccc att gcc cgt ttc tat tcg     384
   Ser Phe Pro Leu His Val Val Lys Asn Pro Ile Ala Arg Phe Tyr Ser
           115                 120                 125
   gtt ttc gcg ctt atc gac gaa gcc tac gca gtc act gcg gcc agg ccc     432
   Val Phe Ala Leu Ile Asp Glu Ala Tyr Ala Val Thr Ala Ala Arg Pro
       130                 135                 140
   gca ggc tgg tcg gcg tgg cga ctt atc tca atg caa ata gcg ttt cac     480
   Ala Gly Trp Ser Ala Trp Arg Leu Ile Ser Met Gln Ile Ala Phe His
   145                 150                 155                 160
   tcc tac tgg gta ttc ggc ggt ctc acc gga gtg gcg atc gca gag ttg     528
   Ser Tyr Trp Val Phe Gly Gly Leu Thr Gly Val Ala Ile Ala Glu Leu
                   165                 170                 175
   att cct ttt gaa att aag ggc ctc gag ttc gcc ctt tgc tct ctc ttt     576
   Ile Pro Phe Glu Ile Lys Gly Leu Glu Phe Ala Leu Cys Ser Leu Phe
               180                 185                 190
   gtc acg ctg act ttg gat tcc tgc cga acg aaa aag cag atc cct tct     624
   Val Thr Leu Thr Leu Asp Ser Cys Arg Thr Lys Lys Gln Ile Pro Ser
           195                 200                 205
   ctg ctg ctc gca ggt ttg agc ttc acc att gct ctt gtg gta att cca     672
   Leu Leu Leu Ala Gly Leu Ser Phe Thr Ile Ala Leu Val Val Ile Pro
       210                 215                 220
   ggt cag gcc cta ttt gcg gcg ctg ctg atc ttc ttg ggt ctg ttg acc     720
   Gly Gln Ala Leu Phe Ala Ala Leu Leu Ile Phe Leu Gly Leu Leu Thr
   225                 230                 235                 240
   atc cgg tac ttc ttc ttg gga aag gct gct aaa                         753
   Ile Arg Tyr Phe Phe Leu Gly Lys Ala Ala Lys
                   245                 250
<210> 3
   <211> 251
   <212> PRT
   <213> Corynebacterium glutamicum ATCC14752
<400> 3
   Val Gln Lys Thr Gln Glu Ile His Ser Ser Leu Glu Val Ser Pro Ser
    1               5                  10                  15
   Lys Ala Ala Leu Glu Pro Asp Asp Lys Gly Tyr Arg Arg Tyr Glu Ile
               20                  25                  30
   Ala Gln Gly Leu Lys Thr Ser Leu Ala Ala Gly Leu Gly Met Tyr Pro
           35                  40                  45
   Ile Gly Ile Ala Phe Gly Leu Leu Val Ile Gln Tyr Gly Tyr Glu Trp
       50                  55                  60
   Trp Ala Ala Pro Leu Phe Ser Gly Leu Ile Phe Ala Gly Ser Thr Glu
   65                  70                  75                  80
   Met Leu Val Ile Ala Leu Val Val Gly Ala Ala Pro Leu Gly Ala Ile
                   85                  90                  95
   Ala Leu Thr Thr Leu Leu Val Asn Phe Arg His Val Phe Tyr Ala Phe
               100                 105                 110
   Ser Phe Pro Leu His Val Val Lys Asn Pro Ile Ala Arg Phe Tyr Ser
           115                 120                 125
   Val Phe Ala Leu Ile Asp Glu Ala Tyr Ala Val Thr Ala Ala Arg Pro
       130                 135                 140
   Ala Gly Trp Ser Ala Trp Arg Leu Ile Ser Met Gln Ile Ala Phe His
   145                 150                 155                 160
   Ser Tyr Trp Val Phe Gly Gly Leu Thr Gly Val Ala Ile Ala Glu Leu
                   165                 170                 175
   Ile Pro Phe Glu Ile Lys Gly Leu Glu Phe Ala Leu Cys Ser Leu Phe
               180                 185                 190
   Val Thr Leu Thr Leu Asp Ser Cys Arg Thr Lys Lys Gln Ile Pro Ser
           195                 200                 205
   Leu Leu Leu Ala Gly Leu Ser Phe Thr Ile Ala Leu Val Val Ile Pro
       210                 215                 220
   Gly Gln Ala Leu Phe Ala Ala Leu Leu Ile Phe Leu Gly Leu Leu Thr
   225                 230                 235                 240
   Ile Arg Tyr Phe Phe Leu Gly Lys Ala Ala Lys
                   245                 250
<210> 4
   <211> 324
   <212> DNA
   <213> Corynebacterium glutamicum ATCC14752
<220>
   <221> CDS
   <222> (1)..(324)
   <223> brnE
<400> 4
   atg aca act gat ttc tcc tgt att ctc ctt gtt gtc gca gta tgt gca      48
   Met Thr Thr Asp Phe Ser Cys Ile Leu Leu Val Val Ala Val Cys Ala
    1               5                  10                  15
   gtc att act ttt gcg ctc cgg gcg gtt ccg ttc tta atc ctt aag ccc      96
   Val Ile Thr Phe Ala Leu Arg Ala Val Pro Phe Leu Ile Leu Lys Pro
               20                  25                  30
   cta cgt gaa tca caa ttt gtg ggc aaa atg gcg atg tgg atg cca gca     144
   Leu Arg Glu Ser Gln Phe Val Gly Lys Met Ala Met Trp Met Pro Ala
           35                  40                  45
   gga atc ctt gcc att ttg acc gca tca acg ttt cgc agc aat gcg ata     192
   Gly Ile Leu Ala Ile Leu Thr Ala Ser Thr Phe Arg Ser Asn Ala Ile
       50                  55                  60
   gat ctg aag act cta acc ttt ggt ctc att gcc gtt gcg att aca gtg     240
   Asp Leu Lys Thr Leu Thr Phe Gly Leu Ile Ala Val Ala Ile Thr Val
   65                  70                  75                  80
   gtg gcg cat ctt ctt ggc ggt cga cgc acc ttg ttg agc gtt ggc gct     288
   Val Ala His Leu Leu Gly Gly Arg Arg Thr Leu Leu Ser Val Gly Ala
                   85                  90                  95
   ggc acc atc gtt ttt gtt gga ctg gtg aat ctt ttc                     324
   Gly Thr Ile Val Phe Val Gly Leu Val Asn Leu Phe
               100                 105
<210> 5
   <211> 108
   <212> PRT
   <213> Corynebacterium glutamicum ATCC14752
<400> 5
   Met Thr Thr Asp Phe Ser Cys Ile Leu Leu Val Val Ala Val Cys Ala
    1               5                  10                  15
   Val Ile Thr Phe Ala Leu Arg Ala Val Pro Phe Leu Ile Leu Lys Pro
               20                  25                  30
   Leu Arg Glu Ser Gln Phe Val Gly Lys Met Ala Met Trp Met Pro Ala
           35                  40                  45
   Gly Ile Leu Ala Ile Leu Thr Ala Ser Thr Phe Arg Ser Asn Ala Ile
       50                  55                  60
   Asp Leu Lys Thr Leu Thr Phe Gly Leu Ile Ala Val Ala Ile Thr Val
   65                  70                  75                  80
   Val Ala His Leu Leu Gly Gly Arg Arg Thr Leu Leu Ser Val Gly Ala
                   85                  90                  95
   Gly Thr Ile Val Phe Val Gly Leu Val Asn Leu Phe
               100                 105
<210> 6
   <211> 1271
   <212> DNA
   <213> Corynebacterium glutamicum ATCC13032
<220>
   <221> gene
   <222> (101)..(853)
   <223> brnF
<220>
   <221> gene
   <222> (853)..(1176)
   <223> brnE
<400> 6
   gcgcgatcaa tggaatctag cttcatatat tgcacaatag cctagttgag gtgcgcaaac 60
   tggcaacaaa actacccggc aattgtgtga tgattgtagt gtgcaaaaaa cgcaagagat 120
   tcattcaagc ctggaggtgt cgccatccaa ggcagccctg gaaccagatg ataaaggtta 180
   tcggcgctac gaaatcgcgc aaggtctaaa aacctccctt gctgcaggtt tgggcatgta 240
   cccgattggt attgcgtttg gtctcttggt tattcaatac ggctacgaat ggtgggcagc 300
   cccactgttt tccggcctga ttttcgcggg ctccaccgaa atgctggtca tcgccctcgt 360
   tgtgggcgca gcgcccctgg gcgccatcgc gctcaccaca ttgctggtga acttccgcca 420
   cgtattctat gcgttttcat tcccgctgca tgtggtcaaa aaccccattg cccgtttcta 480
   ttcggttttc gcgcttatcg acgaagccta cgcagtcact gcggccaggc ccgcaggctg 540
   gtcggcgtgg cgacttatct caatgcaaat agcgtttcac tcctactggg tattcggcgg 600
   tctcaccgga gtggcgatcg cagagttgat tccttttgaa attaagggcc tcgagttcgc 660
   cctttgctct ctctttgtca cgctgacttt ggattcctgc cgaacgaaaa agcagatccc 720
   ttctctgctg ctcgcaggtt tgagcttcac cattgctctt gtggtaattc caggtcaggc 780
   cctatttgcg gcgctgctga tcttcttggg tctgttgacc atccggtact tcttcttggg 840
   aaaggctgct aaatgacaac tgatttctcc tgtattctcc ttgttgtcgc agtatgtgca 900
   gtcattactt ttgcgctccg ggcggttccg ttcttaatcc ttaagcccct acgtgaatca 960
   caatttgtgg gcaaaatggc gatgtggatg ccagcaggaa tccttgccat tttgaccgca 1020
   tcaacgtttc gcagcaatgc gatagatctg aagactctaa cctttggtct cattgccgtt 1080
   gcgattacag tggtggcgca tcttcttggc ggtcgacgca ccttgttgag cgttggcgct 1140
   ggcaccatcg tttttgttgg actggtgaat cttttctaaa actgcataaa taacaaaaat 1200
   ccgcatgccc tcaatttgaa ggggatgcgg attttttaag gaacctagaa aaggcttaag 1260
   cagacagcgc t                                                      1271

## Beschreibung

Gegenstand der Erfindung sind für den Export verzweigtkettiger Aminosäuren kodierende Nukleotidsequenzen, Verfahren zu deren Auffinden und Isolieren und Verfahren zur fermentativen Herstellung von verzweigtkettigen Aminosäuren unter Verwendung von coryneformen Bakterien, in denen Gene, die für den Export verzweigtkettiger Aminosäuren kodieren, über exprimiert werden.

### Stand der Technik

Die verzweigtkettigen Aminosäuren L-Isoleucin, L-Valin, und L-Leucin finden in der pharmazeutischen Industrie, der Humanmedizin, und in der Tierernährung Anwendung.

Es ist bekannt, daß verzweigtkettige Aminosäuren durch Fermentation von Stämmen coryneformer Bakterien, insbesondere Corynebacterium glutamicum hergestellt werden können. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Isoleucin-Analogon Isoleucinhydroxamat (Kisumi M, Komatsubara S, Sugiura, M, Chibata I (1972) Journal of Bacteriology 110: 761-763), das Valin-Analogon 2-Thiazolealanine (Tsuchida T, Yoshinanga F, Kubota K, Momose H (1975) Agricultural and Biological Chemistry, Japan 39: 1319-1322), oder das Leucin-Analogon α-Aminobutyrate (Ambe-Ono Y, Sato K, Totsuka K, Yoshihara Y, Nakamori S (1996) Bioscience Biotechnology Biochemistry 60: 1386-1387) sind, oder die auxotroph für regulatorisch bedeutsame Metabolite sind und verzweigtkettige Aminosäuren produzieren (Tsuchida T, Yoshinaga F, Kubota K, Momose H, Okumura S (1975) Agricultural and Biological Chemistry; Nakayama K, Kitada S, Kinoshita S (1961) Journal of General and Applied Microbiology, Japan 7: 52-69; Nakayama K, Kitada S, Sato Z, Kinoshita (191) Journal General and Applied Microbiology, Japan 7: 41-51).

Seit einigen Jahren werden ebenfalls Methoden der rekombinanten DNA-Technik zur Stammverbesserung verzweigtkettige Aminosäuren produzierender Stämme von Corynebacterium eingesetzt, indem man einzelne verzweigtkettige Aminosäuren-Biosynthesegene amplifiziert, und die Auswirkung auf die verzweigtkettige Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)), Sahm et al. (Annuals of the New York Academy of Science 782, 25-39 (1996)), und Eggeling et al., Journal of Biotechnology 56: 168-180 (1997)).

In der DE 195 48 222 A wird das Corynebacterium glutamicum lysE-Gen, welches für ein Lysin-Exporter-Protein kodiert, offenbart, sowie ein Verfahren zur Herstellung von Aminosäuren, insbesondere von L-Lysin.

Von Eggeling et al.: "The fruits of molecular physiology: engineering the L-isoleucine biosynthesis pathway in Corynebacterium glutamicum" Journal of Biotechnology, Bd. 56, Nr. 3, 28. August 1997, Seiten 167-182, wird zwar die Existenz eines Isoleucin-Exporters vermutet und die Verbesserung Isoleucin-produzierender Corynebacterium glutamicum Stämme durch Erhöhung des Isoleucin-Exports vorgeschlagen, jedoch ist das Exporter-Protein an sich nicht beschrieben.

Hermann T. et al: "Mechanism and Regulation of isoleucine excretion in Corynebacterium glutamicum" Applied and Environmental Microbiology, Bd. 62, Nr. 9, September 1996, Seiten 3238-3244 erwähnen zwar, dass Aminosäure-haltige Peptide in C. glutamicum nicht katabolisiert werden und somit für ein Wachstum des Bakteriums auf Paptid-haltigem Medium exportiert werden müssen. Sie beschreiben jedoch nicht explizit, dass im Isoleucin-Export defekte Corynebakterien dem Isoleucin-enthaltende Peptide zugesetzt wurden, schlechtes Wachstum zeigen.

### Aufgabe der Erfindung

Die Erfinder haben sich zur Aufgabe gestellt, neue Maßnahmen zur verbesserten fermentativen Herstellung von verzweigtkettigen Aminosäuren bereitzustellen.

### Beschreibung der Erfindung

Verzweigtkettige Aminosäuren finden in der pharmazeutischen Industrie, in der Humanmedizin, und in der Tierernährung Anwendung. Es besteht daher ein allgemeines Interesse daran neue verbesserte Verfahren zur Herstellung von verzweigtkettigen Aminosäuren bereitzustellen.

Wenn im folgenden verzweigtkettige Aminosäuren erwähnt werden sind damit insbesondere L-Isoleucin, L-Valin oder L-Leucin gemeint.

Gegenstand der Beschreibung sind isolierte Polynukleotide, enthaltend mindestens eine der Polynukleotidsequenzen, ausgewählt aus der Gruppe
a) Polynukleotid, das zu mindestens 70 % identisch ist mit einem Polynukleotid, das für ein Polypeptid codiert, das mindesten eine Aminosäuresequenz SEQ ID No. 3 oder 5 enthält,
b) Polynukleotid, das für ein Polypeptid codiert, das eine Aminosäuresequenz enthält, die zu mindestens 70 % identisch ist mit einer Aminosäuresequenz von SEQ ID No. 3 oder 5,
c) Polynukleotid, das komplementär ist zu den Polynukleotiden von a) oder b), und
d) Polynukleotid, enthaltend mindestens 15 aufeinanderfolgende Basen der Polynukleotidsequenzenen von a), b) oder c).

Gegenstand der Beschreibung ist ebenso eine in coryneformen Mikroorganismen replizierbare, bevorzugt rekombinante DNA mit der Herkunft Corynebacterium, die zumindest die Nukleotidsequenzen enthält, die für die Gene brnF und/oder brnE, dargestellt in der SEQ ID No. 1 und in der SEQ ID No. 6, kodieren.

Gegenstand ist ebenfalls eine replizierbare DNA, enthaltend:
(i) die Nukleotidsequenzen, gezeigt in SEQ-ID-No. 1 oder SEQ-ID-No. 6, die für die Gene brnE und/oder brnF codieren, oder
(ii) mindestens eine Sequenz, die den Sequenzen (i) innerhalb des Bereichs der Degeneration des genetischen Codes entspricht, oder
(iii) mindestens eine Sequenz, die mit den zu den Sequenzen (i), oder(ii) komplementären Sequenz hybridisiert, und gegebenenfalls
(iv) funktionsneutralen Sinnmutationen in (i).

### Weitere Gegenstände sind

Polynukleotide, enthaltend mindestens eine der Nukleotidsequenzen, ausgewählt aus den SEQ ID No. 1, 2, 4 oder 6,
Polynukleotide, die für Polypeptide codieren, die mindestens eine der Aminosäuresequenzen, wie in SEQ ID No. 3 oder 5 dargestellt, enthalten, ein Vektor, enthaltend das oder die Polynukleotide gemäß
Anspruch 1, oder die in SEQ ID No. 1 oder SEQ ID No. 6 dargestellte DNA-Sequenz. und als Wirtszelle dienende coryneforme Bakterien, die den Vektor enthalten.

Gegenstand der Erfindung sind gemäß den Ansprüchen 20 bis 29 :
Isolierte Polynukleotide,
   ausgewählt aus der Gruppe
   a) Polynukleotid, das für ein Polypeptid codiert, welches die Funktion eines Exportproteins für eine verzweigtkettige Aminosäure und die Aminosäuresequenz von SEQ ID No. 3 oder 5 besitzt,
   b) Polynukleotid, das für ein Polypeptid codiert, welches die Funktion eines Exportproteins für eine verzweigtkettige Aminosäure eine Aminosäuresequenz besitzt, die zu mindestens 90 % identisch ist mit der Aminosäuresequenz von SEQ ID No. 3 oder 5, und
   c) Polynukleotid, das komplementär ist zu den Polynukleotiden von a) oder b).

Polynukleotid gemäß Anspruch 20 mit der Nukleotidsequenz SEQ ID No. 2,

Polynukleotid gemäß Anspruch 20 die für die Aminosäuresequenz mit der SEQ ID No. 5 kodiert,

Polynukleotid gemäß Anspruch 20 mit der Nukleotidsequenz SEQ ID No. 4 ,
Polynukleotid gemäß einem oder mehreren der Ansprüche 20 bis 24, mit der Nukleotidsequenz von SEQ ID No. 1 oder 6,
Vektoren, die ein Polynukleotid gemäß einem oder mehreren der Ansprüche 20 bis 24 enthalten,
Coryneforme Bakterien oder Escherichia coli, die den Vektor gemäß Anspruch 25 enthalten,
Coryneforme Bakterien, die das isolierte Polynukleotid gemäß einem der Ansprüchen 20 bis 24 in ihrem Chromosom amplifiziert enthalten sind Teil der Beschreibung,
Coryneforme Bakterien gemäß Anspruch 26, **dadurch gekennzeichnet, dass** es sich um Corynebacterium glutamicum handelt,
Coryneforme Bakterien gemäß einem oder mehreren der Ansprüche 26 bis 27, **dadurch gekennzeichnet, dass** es sich um einen Stamm handelt, der eine verzweigtkettige Aminosäure produziert,
Oligonukleotid-Primer oder Oligonukleotid-Sonde, enthaltend mindestens 15 aufeinanderfolgende Nukleotide, ausgewählt aus SEQ ID No.4 oder der dazu komplementären Nukleotidsequenz.

Gegenstand der Beschreibung sind ebenso Polynukleotide, die im wesentlichen aus einer Polynukleotidsequenz bestehen, die erhältlich sind durch Screening mittels Hybridisierung einer entsprechenden Genbank, die die vollständigen Gene mit den Polynukleotidsequenzen entsprechend SEQ ID No. 1, 2, 4 oder 6 enthalten, mit einer Sonde, die die Sequenzen der genannten Polynukleotide gemäß SEQ ID No 1, 2, 4 oder 6 oder ein Fragment davon enthalten und Isolierung der genannten DNA-Sequenzen.

Polynukleotidsequenzen gemäß der Beschreibung sind geeignet, als Hybridisierungs-Sonden für RNA, cDNA und DNA, um cDNA in voller Länge zu isolieren, die für Isoleucin-, Leucin-oder Valin-Exportproteine codieren und solche cDNA oder Gene zu isolieren, die eine hohe Ähnlichkeit der Sequenz mit der des brnF- und/oder brnE-Gens aufweisen.

Polynukleotidsequenzen gemäß der Beschreibung sind weiterhin als Primer geeignet, mit deren Hilfe mit der Polymerase Kettenreaktion (PCR) DNA von Genen hergestellt werden kann, die für Isoleucin-, Leucin- oder Valin-Exportproteine codieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Basenpaaren.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäure enthalten.

Die Polypeptide gemäß der Beschreibung besitzen die Aminosäuresequenz gemäß SEQ ID No. 3 und/oder 5, und die biologische Aktivität des Transports verzweigtkettigter Aminosäuren. Dazu gehören auch solche, die zu wenigstens 70 % identisch sind mit den Polypeptiden gemäß SEQ ID No. 3 und/oder 5, bevorzugt zu wenigstens 80 % und besonders zu wenigstens 90 % bis 95 % Identität mit den Polypeptiden gemäß SEQ ID No. 3 und/oder 5 und die genannte Aktivität aufweisen.

Ebenso beschrieben sind coryneforme Mikroorganismen, insbesondere der Gattung Corynebacterium, transformiert durch die Einführung eines Vektors gemäß Anspruch 25.

Die Erfindung betrifft weiter ein Verfahren zur fermentativen Herstellung von verzweigtkettigen Aminosäuren unter Verwendung von coryneformen Bakterien, die insbesondere bereits die verzweigtkettigen Aminosäuren produzieren und in denen die Nukleotidsequenzen der für den Export verzweigtkettiger Aminosäuren kodierenden Gene brnE und/oder brnF überexprimiert werden.

Der Begriff "Verstärkung" beschreibt in diesem Zusammenhang die Erhöhung der intrazellulären Aktivität eines oder mehrerer Enzyme (Proteine) in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym (Protein) mit einer hohen Aktivität kodiert und gegebenenfalls diese Maßnahmen kombiniert.

Die Mikroorganismen, die Gegenstand der vorliegenden Erfindung sind, können verzweigtkettige Aminosäuren aus Glucose, Saccharose, Lactose, Mannose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind beispielsweise die bekannten Wildtypstämme
Corynebacterium glutamicum ATCC13032
Brevibacterium flavum ATCC14067
Brevibacterium lactofermentum ATCC13869 und
Brevibacterium divaricatum ATCC14020
   und daraus hergestellte verzweigtkettige Aminosäuren produzierende Mutanten bzw. Stämme,

wie beispielsweise die Isoleucin produzierenden Stämme
Corynebacterium glutamicum ATCC 14309
Corynebacterium glutamicum ATCC 14310
Corynebacterium glutamicum ATCC 14311
Corynebacterium glutamicum ATCC 15168
Corynebacterium ammoniagenes ATCC 6871,
   wie beispielsweise die Leucin produzierenden Stämme
   Corynebacterium glutamicum ATCC 21885
   Brevibacterium flavum ATCC 21889
      oder wie beispielsweise die Valin produzierenden Stämme Corynebacterium glutamicum DSM 12455 Corynebacterium glutamicum FERM-P 9325 Brevibacterium lactofermentum FERM-P 9324 Brevibacterium lactofermentum FERM-BP 1763.

Den Erfindern gelang es die neuen Gene brnE und brnF von Corynebacterium glutamicum zu isolieren. Zur Isolierung der Gene wird zunächst eine im brnF- oder brnE-Gen defekte Mutante von C. glutamicum hergestellt. Hierzu wird ein geeigneter Ausgangsstamm wie z. B. ATCC14752 oder ATCC13032 einem Mutagenese-Verfahren unterworfen.

Klassische Mutagenese-Verfahren sind die Behandlung mit Chemikalien wie z. B. N-Methyl-N-Nitro-N-Nitrosoguanidin oder UV-Bestrahlung. Derartige Verfahren zur Mutationsauslösung sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Ein anderes Mutagenese-Verfahren ist die Methode der Transposonmutagenese bei der die Eigenschaft eines Transposons ausgenutzt wird in DNA-Sequenzen zu "springen" und dadurch die Funktion des betreffenden Gens zu stören bzw. auszuschalten. Transposons coryneformer Bakterien sind in der Fachwelt bekannt. So wurden aus Corynebacterium xerosis Stamm M82B das Erythromycinresistenz-Transposon Tn5432 (Tauch et al., Plasmid (1995) 33: 168-179) und das Chloramphenicolreistenz-Transposon Tn5546 isoliert. Tauch et al. (Plasmid (1995) 34: 119-131 und Plasmid (1998) 40: 126-139) zeigten, daß eine Mutagenese mit diesen Transposonen möglich ist.

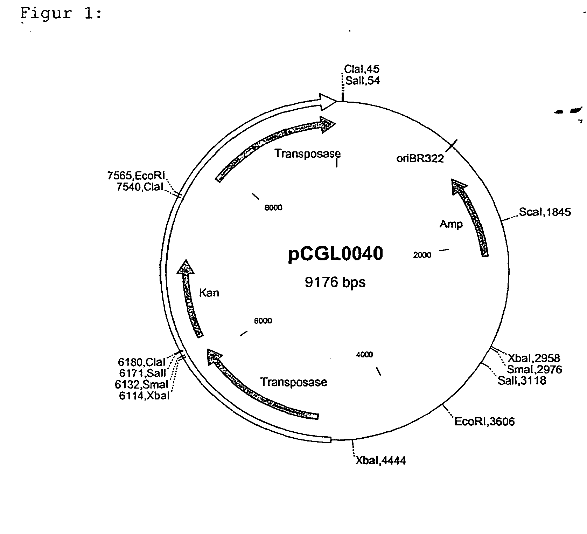

Ein anderes Transposon ist das Transposon Tn5531 das bei Ankri et al. (Journal of Bacteriology (1996) 178: 4412-4419) beschrieben wird und beispielhaft im Laufe der vorliegenden Erfindung eingesetzt wurde. Das Transposon Tn5531 enthält das aph3 Kanamycinresistenzgen und kann in Form des Plasmidvektors pCGL0040 verabreicht werden, der in Figur 1 dargestellt ist. Die Nukleotidsequenz des Tansposons Tn5531 ist unter der Zugangsnummer (accession number) U53587 bei dem National Center for Biotechnology Information (NCBI, Bethesda, MD, USA) frei verfügbar.

Nach erfolgter Mutagenese vorzugsweise Transposon-Mutagenese wird eine im brnF- oder brnE-Gen defekte Mutante gesucht. Eine im brnF- oder brnE-Gen defekte Mutante wird daran erkannt, daß sie auf Minimalagar gutes Wachstum aber auf Minimalagar, der mit verzweigtkettigen Aminosäurehaltigen Oligopeptiden wie z. B. dem Dipeptid Isoleucyl-isoleucin supplementiert wurde, schlechtes Wachstum zeigt.

Ein Beispiel für eine derartige Mutante ist der Stamm ATCC14752brnE::Tn5531.

Ein auf die beschriebene Weise hergestellter Stamm kann dann für die Klonierung und Sequenzierung des brnF-und/oder brnE-Gens verwendet werden.

Hierzu kann eine Genbank des interessierenden Bakteriums angelegt werden. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Für die vorliegende Erfindung eignen sich solche Vektoren, die in coryneformen Bakterien vorzugsweise Corynebacterium glutamicum replizieren. Derartige Vektoren sind aus dem Stand der Technik bekannt; als Beispiel sei der Plasmidvektor pZ1 genannt, der bei Menkel et al. (Applied and Environmental Microbiology (1989) 64: 549-554) beschrieben ist. Die auf die beschriebene Weise erhaltene Genbank wird anschließend mittels Transformation oder Elektroporation in den im brnF- oder brnE-Gen defekten Indikatorstamm überführt und solche Transformanten gesucht, die die Fähigkeit besitzen in Gegenwart verzweigtkettiger Aminosäure-haltiger Oligopeptide auf Minimalagar zu wachsen. Das klonierte DNA-Fragment kann anschließend einer Sequenzanalyse unterzogen werden.

Bei Verwendung einer durch Tn5531-Mutagenese erzeugten Mutante eines coryneformen Bakteriums wie z. B. dem Stamm ATCC14752brnE::Tn5531 kann das brnE::Tn5531-Allel direkt unter Ausnutzung des in ihm enthaltenen Kanamycinresistenzgens aph3 kloniert und isoliert werden. Hierzu verwendet man bekannte Kloniervektoren wie z. B. pUC18 (Norrander et al., Gene (1983) 26: 101-106 und Yanisch-Perron et al., Gene (1985) 33: 103-119). Als Klonierwirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die Selektion auf Transformanten erfolgt in Gegenwart von Kanamycin. Die Plasmid-DNA der erhaltenen Transformanten wird anschließend sequenziert. Hierzu kann die von Sanger et al. (Proceedings of the National Academy of Sciences of the United States of America USA (1977) 74: 5463-5467) beschriebene Dideoxy-Kettenabbruchmethode verwendet werden. Hiernach erhält man die stromaufwärts und stromabwärts des Tn5531-Insertionsortes enthaltenen Gene. Die erhaltenen Nukleotidsequenzen werden dann mit kommerziell erhältlichen Sequenzanalyse-Programmen wie z. B.dem Programmpaket Lasergene (Biocomputing Software for Windows, DNASTAR, Madison, USA) oder dem Programmpaket HUSAR (Release 4.0, EMBL, Heidelberg, Deutschland) analysiert und zusammengefügt.

Auf diese Weise wurden die neuen für den Export verzweigtkettiger Aminosäuren kodierenden DNA-Sequenzen von C. glutamicum erhalten, die als SEQ ID NO 1, Bestandteil der vorliegenden Erfindung sind. In SEQ ID NO 2 und SEQ ID NO 4 sind die Kodierregionen des brnF- und brnE-Gens dargestellt. In SEQ ID NO 3 und SEQ ID NO 5 sind die Aminosäuresequenzen der sich aus SEQ ID NO 1, bzw. SEQ ID NO 2 und SEQ ID NO 4 ergebenden Genprodukte dargestellt.

Kodierende DNA-Sequenzen, die sich aus SEQ ID NO 1 durch die Degeneriertheit des genetischen Codes ergeben, sind ebenfalls Bestandteil der Beschreibung. In gleicher Weise sind DNA-Sequenzen, die mit SEQ ID NO 1, oder Teilen von SEQ ID NO 1 hybridisieren, Bestandteil der Beschreibung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 oder SEQ ID NO 4 ergeben sind ebenfalls Bestandteil der Erfindung.

Unter Ausnutzung der in SEQ-ID-No. 1 dargestellten Nukleotidsequenz können geeignete Primer synthetisiert und diese dann dazu verwendet werden mit Hilfe der Polymerase-Kettenreaktion (PCR) brnF-, und brnE-Gene verschiedener coryneformer Bakterien und Stämme zu amplifizieren. Anleitungen hierzu findet der Fachmann unter anderem beispielsweise im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994). Alternativ kann die in SEQ-ID-No. 1 dargestellte Nukleotidsequenz oder Teile davon als Sonde zur Suche von brnF- und/oder brnE-Genen in Genbanken von insbesondere coryneformen Bakterien verwendet werden. Anleitungen hierzu findet der Fachmann unter anderem beispielsweise im Handbuch "The DIG System Users Guide for Filter Hybridization" der Firma Firma Roche Diagnostics (Mannheim, Deutschland) und bei Liebl et al. (International Journal of Systematic Bacteriology (1991) 41: 255-260). Die auf diese Weise amplifzierten brnE-, und brnF-Gene enthaltenden DNA-Fragmente werden anschließend kloniert und sequenziert.

Auf diese Weise wurde die in SEQ-ID-No. 6 dargestellte DNA-Sequenz der Gene brnF und brnE des Stammes ATCC13032 erhalten, die ebenfalls Bestandteil der vorliegenden Erfindung ist.

Die Erfinder fanden heraus, daß coryneforme Bakterien nach Überexpression des brnF- und/oder brnE Export-Gens in verbesserter Weise verzweigtkettige Aminosäuren produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen Herstellung verzweigtkettiger Aminosäuren zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der EP-B 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurden die erfindungsgemäßen Gene brnF und brnE mit Hilfe von Plasmiden überexprimiert. Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Zusätzlich kann es für die Produktion von verzweigtkettigen Aminosäuren vorteilhaft sein, neben den neuen brnF- und brnE-Genen ein oder mehrere für weitere Enzyme des bekannten Biosyntheseweges der verzweigtkettigen Aminosäuren oder Enzyme des anaplerotischen Stoffwechsels oder Enzyme des Zitronensäure-Zyklus kodierende Gene zu überexprimieren.

So kann beispielsweise zur Produktion von L-Isoleucin
- gleichzeitig das für die Homoserin-Dehydrogenase kodierende hom-Gen (Peoples et al., Molecular Microbiology 2, 63-72 (1988)) oder das für eine "feed back resistente" Homoserin-Dehydrogenase kodierende hom^{dr}-Allel (Archer et al., Gene 107, 53-59 (1991)) oder
- gleichzeitig das für die Threonin-Dehydratase kodierende ilvA-Gen (Möckel et al., Journal of Bacteriology (1992) 8065-8072)) oder das für eine "feed back resistente" Threonin- Dehydratase kodierende ilvA(Fbr)-Allel (Möckel et al., (1994) Molecular Microbiology 13: 833-842), oder
- gleichzeitig die für die Acetohydroxysäuresynthase kodierenden Gene ilvBN (Keilhauer et al., (1993) Journal of Bacteriology 175: 5595-5603), oder
- gleichzeitig das für die Dihydroxysäuredehydratase kodierende ilvD-Gen (Sahm und Eggeling (1999) Applied and Environmental Microbiology 65: 1973-1979), oder
- gleichzeitig das für die Pyruvat-Carboxylase kodierende pyc-Gen (DE-A-19 831 609), oder
- gleichzeitig das für die Malat:Chinon Oxidoreduktase kodierende mqo-Gen (Molenaar et al., European Journal of Biochemistry 254, 395 - 403 (1998))
überexprimiert werden.

So kann beispielsweise zur Produktion von L-Leucin,
- gleichzeitig das für die Isopropylmalatsynthase kodierende leuA-Gen (Pátek et al., Applied Environmental Microbiology 60 (1994) 133-140) oder ein für eine "feed back resistente" Isopropylmalatsynthase kodierendes Allel, oder
- gleichzeitig die für die Isopropylmalatdehydratase kodierenden leuC- und leuD-Gene (Pátek et al., Applied Environmental Microbiology 60 (1994) 133-140), oder
- gleichzeitig das für die Isopropylmalatdehydrogenase kodierenden leuB-Gen (Pátek et al., Applied Environmental Microbiology 60 (1994) 133-140), oder
- gleichzeitig die für die Acetohydroxysäuresynthase kodierenden Gene ilvBN (Keilhauer et al., (1993) Journal of Bacteriology 175: 5595-5603), oder
- gleichzeitig das für die Dihydroxysäuredehydratase kodierende ilvD-Gen (Sahm und Eggeling (1999) Applied and Environmental Microbiology 65: 1973-1979), oder
- gleichzeitig das für die Malat:Chinon Oxidoreduktase kodierende mqo-Gen (Molenaar et al., European Journal of Biochemistry 254, 395 - 403 (1998))
   überexprimiert werden.

So kann beispielsweise zur Produktion von L-Valin
- gleichzeitig die für die Acetohydroxysäuresynthase kodierenden Gene ilvBN (Keilhauer et al., (1993) Journal of Bacteriology 175: 5595-5603), oder
- gleichzeitig das für die Dihydroxysäuredehydratase kodierende ilvD-Gen (Sahm und Eggeling (1999) Applied and Environmental Microbiology 65: 1973-1979), oder
- gleichzeitig das für die Malat:Chinon Oxidoreduktase kodierende mqo-Gen (Molenaar et al., European Journal of Biochemistry 254, 395 - 403 (1998))
   überexprimiert werden.

Weiterhin kann es für die Produktion von verzweigtkettigen Aminosäuren vorteilhaft sein, neben der Überexpression des brnE- und/oder brnF-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion verzweigtkettiger Aminosäuren kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) erhalten.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden. Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an verzweigtkettigen Aminosäuren gebildet hat. Dieses Ziel wird normalerweise innerhalb von 10 Stunden bis 160 Stunden erreicht.

Die Analyse der verzweigtkettigen Aminosäuren kann durch Anionenaustauschchromatographie mit anschließender Ninhydrin Derivatisierung erfolgen, so wie bei Spackman et al. (Analytical Chemistry, 30, (1958), 1190) beschrieben, oder sie kann durch reversed phase HPLC erfolgen so wie bei Lindroth et al. (Analytical Chemistry (1979) 51: 1167-1174) beschrieben.

Folgender Mikroorganismus wurden bei der Deutschen Sammlung für Mikrorganismen und Zellkulturen (DSMZ, Braunschweig, Deutschland) gemäß Budapester Vertrag hinterlegt:
- Escherichia coli Stamm GM2929pCGL0040 als DSM 12839

### Beispiele

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular cloning. A laboratory manual (1989) Cold Spring Harbour Laboratory Press) durchgeführt. Die Transformation von Escherichia coli wurde, wenn nicht anders beschrieben, nach Chung et al. (Proceedings of the National Academy of Sciences of the United States of America (1989) 86: 2172-2175) durchgeführt.

### Beispiel 1

Klonierung und Sequenzierung des brnF- und brnE-Gens von Corynebacterium glutamicum ATCC14752

### 1.Transposonmutagenese

Der Stamm Corynebacterium glutamicum ATCC14752 wurde einer Mutagenese mit dem Transposon Tn5531 unterworfen, dessen Sequenz unter der Accession-Nummer U53587 in der Nukleotid-Datenbank des National Center for Biotechnology Information (Bethesda, USA) hinterlegt ist. Aus dem Methylase-defekten E. coli-Stamm GM2929pCGL0040 (E. coli GM2929: Palmer et al., Gene (1994) 143: 1-12) wurde das Plasmid pCGL0040 isoliert, welches das zusammengesetzte Transposon Tn5531 enthält (Ankri et al., Journal of Bacteriology (1996) 178: 4412-4419). Der Stamm Corynebacterium glutamicum ATCC14752 wurde mittels Elektroporation (Haynes et al., FEMS Microbiology Letters (1989) 61: 329-334) mit dem Plasmid pCGL0040 transformiert. Klone, bei denen das Transposon Tn5531 ins Genom integriert war, wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Auf diese Weise wurden 2000 Klone erhalten, welche auf verzögertes Wachstum in Anwesenheit von Isoleucyl-isoleucin überprüft wurden. Dazu wurden alle Klone einzeln auf CGXII-Minimalmedium-Agarplatten mit und ohne 3 mM Isoleucyl-isoleucin übertragen. Das Medium war identisch mit dem bei Keilhauer et al. beschriebenen Medium CGXII (Journal of Bacteriology (1993) 175: 5593-5603), enthielt aber zusätzlich 25 µg/mL Kanamycin und 15 g/L Agar. Die Zusammensetzung des von Keilhauer et al. beschriebenen Mediums ist in Tabelle 1 dargestellt.

**Tabelle 1**

| | |
|---|---|
| Zusammensetzung des Mediums CGXII | |

| Komponente | Konzentration |
|---|---|
| (NH₄)₂SO₄ | 20 g/L |
| Harnstoff | 5 g/L |
| KH₂PO₄ | 1 g/L |
| K₂HPO₄ | 1 g/L |
| MgSO₄ x 7 H₂O | 0,25 g/L |
| 3-Morpholinopropansulfonsäure | 42 g/L |
| CaCl₂ | 10 mg/L |
| FeSO₄ x 7 H₂O | 10 mg/L |
| MnSO₄ x H₂O | 10 mg/L |
| ZnSO₄ x 7H₂O | 1 mg/L |
| CuSO₄ | 0,2 mg/L |
| NiCl₂ x 6 H₂O | 0,02 mg/L |
| Biotin | 0,2 mg/L |
| Glukose | 40 g/L |
| Protokatechusäure | 30 mg/L |

Die Agarplatten wurden bei 30°C inkubiert und das Wachstum nach 12, 18 und 24 Stunden untersucht. Es wurde eine Transposonmutante erhalten, die ohne Isoleucyl-isoleucin vergleichbar mit dem Ausgangsstamm Corynebacterium glutamicum ATCC14752 wuchs, in Anwesenheit von 3 mM Isoleucyl-isoleucin aber verzögertes Wachstum zeigte. Diese wurde als ATCC14752brnF::Tn5531 bezeichnet.

### 2. Klonierung und Sequenzierung des Insertionsortes von Tn5531 in ATCC14752brnF::Tn5531

Um den stromabwärts des Transposons Tn5531 gelegenen Insertionsort in der in Beispiel 1.1 beschriebenen Mutante zu klonieren, wurde zunächst die chromosomale DNA dieses Mutantenstammes wie bei Schwarzer et al. (Bio/Technology (1990) 9: 84-87) beschrieben isoliert und 400 ng davon mit der Restriktionsendonuklease EcoRI geschnitten. Der vollständige Restriktionsansatz wurde in den ebenfalls mit EcoRI linearisierten Vektor pUC18 (Norander et al., Gene (1983) 26: 101-106) der Firma Roche Diagnostics (Mannheim, Deutschland) ligiert. Mit dem gesamten Ligationsansatz wurde der E. coli Stamm DH5αmcr (Grant et al., Proceedings of the National Academy of Sciences of the United States of America (1990) 87: 4645-4649) mittels Elektroporation transformiert (Dower et al., Nucleic Acid Research (1988) 16: 6127-6145). Transformanten, bei denen auf dem Vektor pUC18 die Insertionsorte des Transposons Tn5531 kloniert vorlagen, wurden anhand ihrer Carbenicillin- und Kanamycinresistenz auf 50 µg/mL Carbenicillin und 25 µg/mL Kanamycin enthaltenden LB-Agarplatten identifiziert. Aus drei der Transformanten wurden die Plasmide präpariert und durch Restriktionsanalyse die Größen der klonierten Inserts bestimmt. Die Nukleotidsequenz des Insertionsortes auf einem der Plasmide mit einem ca. 7,2 kb großen Insert wurde nach der Dideoxy-Kettenabbruchmethode von Sanger et al. bestimmt (Proceedings of the National Academy of Sciences of the United States of America (1977) 74: 5463-5467). Hierzu wurden 1,3 kb des Inserts ausgehend von folgendem Oligonukleotid-Primer sequenziert: 5'-CGG GTC TAC ACC GCT AGC CCA GG-3'.

Zur Identifizierung des stromaufwärts des Transposons gelegenen Insertionsortes wurde die chromosomale DNA der Mutante mit der Restriktionsendonuklease PstI geschnitten und in den mit PstI linearisierten Vektor pUC18 ligiert. Die weitere Klonierung wurde wie oben beschrieben durchgeführt. Die Nukleotidsequenz des Insertionsortes auf einem der Plasmide mit einem ca. 4,8 kb großen Insert wurde nach der Dideoxy-Kettenabbruchmethode von Sanger et al. bestimmt (Proceedings of the National Academy of Sciences of the United States of America (1977) 74: 5463-5467). Hierzu wurden 1,6 kb des Inserts ausgehend von folgendem Oligonukleotid-Primer sequenziert: 5'-CGG TGC CTT ATC CAT TCA GG-3'.

Die erhaltenen Nukleotidsequenzen wurde mit dem Programmpaket Lasergene (Biocomputing Software for Windows, DNASTAR, Madison, USA) analysiert und zusammengefügt. Diese Nukleotidsequenz ist als SEQ ID NO 1 wiedergegeben. Die Analyse ergab die Identifizierung von zwei offenen Leserastern von 753 bp, und 324 bp Länge, die als SEQ ID NO 2 und SEQ ID NO 4 dargestellt sind. Die entsprechenden Gene wurden als brnF- und brnE-Gen bezeichnet. Die dazugehörigen Genprodukte umfassen 251 und 108 Aminosäuren und sind als SEQ ID NO 3 und SEQ ID NO 5 wiedergegeben.

### Beispiel 2

Klonierung und Sequenzierung der brnF-, und brnE-Gene aus Corynebacterium glutamicum ATCC13032

Die Gene brnE und brnF des Stammes ATCC13032 wurden in den E. coli Klonierungsvektor pUC18 (Norrander et al., Gene (1983) 26: 101-106, Roche Diagnostics, Mannheim, Deutschland) kloniert. Die Klonierung wurde in zwei Schritten durchgeführt. Zunächst wurden durch eine Polymerasekettenreaktion (PCR) die Gene aus Corynebacterium glutamicum ATCC13032 mittels folgender aus SEQ ID NO 1 abgeleiteter Oligonukleotid-Primer amplifiziert.

brnE, brnF, -forward:
5'-[AGC GCT GTC TGC TTA AGC CTT TTC]-3'
brnE, brnF, -reverse:
   5'-[GCG CGA TCA ATG GAA TCT AGC TTC]-3'

Die PCR-Reaktion wurde in 30 Zyklen in Gegenwart von 200 µM Deoxynukleotid-triphosphaten (dATP, dCTP, dGTP, dTTP), je 1 - µM des entsprechenden Oligonukleotids, 100 ng chromosomaler DNA von Corynebacterium glutamicum ATCC13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 58°C für 30 Sekunden und 72°C für 2 Minuten.

Das amplifizierte etwa 1,3 kb große Fragment wurde dann im folgenden mit Hilfe des SureClone Ligation Kit (Amersham Pharmacia Biotech, Uppsala, Schweden) nach Angaben des Herstellers in die SmaI-Schnittstelle des Vektors pUC18 ligiert. Mit dem gesamten Ligationsansatz wurde der E. coli Stamm DH5αmcr (Grant et al., Proceedings of the National Academy of Sciences of the United States of America (1990) 87: 4645-4649) transformiert. Transformanten wurden anhand ihrer Carbenicillinresistenz auf 50 µg/mL Carbenicillin enthaltenden LB-Agarplatten identifiziert. Aus 8 der Transformanten wurden die Plasmide präpariert und durch Restriktionsanalyse auf das Vorhandensein des 1,3 kb PCR-Fragments als Insert überprüft. Das so entstandene rekombinante Plasmid wird im folgenden mit pUC18brnEF bezeichnet.

Die Nukleotidsequenz des 1,3 kb PCR-Fragments in Plasmid pUC18brnEF wurde nach der Dideoxy-Kettenabbruchmethode von Sanger et al. durchgeführt (Proceedings of the National Academy of Sciences of the United States of America (1977) 74: 5463-5467). Hierzu wurde das vollständige Insert von pUC18brnEF mit Hilfe folgender Primer der Firma Roche Diagnostics (Mannheim, Deutschland) sequenziert.

Universalprimer:
5'-GTA AAA CGA CGG CCA GT-3'

Reverseprimer:
5'-GGA AAC AGC TAT GAC CAT G-3'

Die erhaltene Nukleotidsequenz ist als SEQ ID NO 6 wiedergegeben. Die erhaltene Nukleotidsequenz wurde mit dem Programmpaket Lasergene (Biocomputing Software for Windows, DNASTAR, Madison, USA) analysiert.

### Beispiel 3

Expression der Gene brnEF aus Corynebacterium glutamicum ATCC13032

Die Gene brnEF wurden in den E. coli - C. glutamicum Shuttle-Vektor pJC1 (Cremer et al., 1990, Molecular and General Genetics 220: 478 - 480) kloniert. Die Klonierung wurde in zwei Schritten durchgeführt. Zunächst wurde durch eine Polymerasekettenreaktion (PCR) das Gen aus Corynebacterium glutamicum ATCC13032 mittels folgender aus SEQ ID NO 6 abgeleiteter Oligonukleotid-Primer amplifiziert.
brnEF-forward:
5'-GCT CTA GAA CCT TGT CAG CCA GTG CG-3'
brnEF-reverse:
   5'-GCT CTA GAA AAA ATC CGC ATC CCC TCA-3'

Diese Oligonukleotid-Primer enthielten zusätzlich an der 5'-Seite eine XbaI-Erkennungsstelle. Die PCR-Reaktion wurde in 30 Zyklen in Gegenwart von 200 µM Deoxynukleotid-triphosphaten (dATP, dCTP, dGTP, dTTP), je 1 µM des entsprechenden Oligonukleotids, 100 ng chromosomaler DNA von Corynebacterium glutamicum ATCC13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 58°C für 30 Sekunden und 72°C für 2 Minuten. Das amplifizierte etwa 1,3 kb große Fragment wurde dann im folgenden mit dem Restriktionsenzym XbaI nach Angaben des Herstellers Roche Diagnostics (Mannheim, Deutschland) behandelt, und mit Hilfe des RapidLigation Kit (Roche Diagnostics) in die XbaI-Schnittstelle des Vektors pJC1 ligiert. Mit dem gesamten Ligationsansatz wurde der E. coli Stamm DH5αmcr (Grant et al., Proceedings of the National Academy of Sciences of the United States of America USA (1990) 87: 4645-4649) transformiert. Transformanten wurden anhand ihrer Kanamycinresistenz auf 50 µg/mL Kanamycin enthaltenden LB-Agarplatten identifiziert. Aus 8 der Transformanten wurden die Plasmide präpariert und durch Restriktionsanalyse auf das Vorhandensein des 1,3 kb PCR-Fragments als Insert überprüft. Das so entstandene rekombinante Plasmid wird im folgenden mit pJC1brnEF bezeichnet. Mittels Elektroporation (Haynes et al., FEMS Microbiology Letters (1989) 61: 329-334) wurden die Plasmide pJC1 und pJC1brnEF in den isoleucinbildenden Stamm Corynebacterium glutamicum DM368-2 eingebracht. Der Stamm DM368-2 ist in der EP-B-0 385 940 beschrieben und als DSM5399 bei der Deutschen Sammlung für Mikroorganismen und Zellkulturen (DSMZ, Braunschweig, Deutschland) gemäß Budapester Vertrag hinterlegt. Transformanten wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Auf diese Weise entstanden die Corynebacterium glutamicum Stämme DM368-2/pJC1 und DM368-2/pJC1brnEF.

### Beispiel 4

Herstellung von L-Isoleucin mit Corynebacterium glutamicum Zur Untersuchung ihrer Isoleucinbildung wurden die Stämme DM368-2/pJC1 und DM368-2/pJC1brnEF in 50 mL Brain Heart Infusion-Medium mit 50 µg Kanamycin/mL (Difco Laboratories, Detroit, USA) für 14 Stunden bei 30°C vorkultiviert. Anschließend wurden die Zellen zweimal mit 0,9%(w/v) Natriumchloridlösung gewaschen und mit dieser Suspension je 60 mL CgXII-Medium so angeimpft, daß die OD₆₀₀ (optische Dichte bei 600 nm) 0,1 betrug. Das Medium war identisch mit dem bei Keilhauer et al. beschriebenen Medium (Journal of Bacteriology (1993) 175: 5593-5603), enthielt aber zusätzlich 50 µg Kanamycin pro mL. Die Kultivierung beider Stämme wurde bei 30°C und 130 rpm durchgeführt. Nach 24 und 48 Stunden wurden jeweils Proben genommen und die Zellen kurz abzentrifugiert (5 Minuten bei 13000 Umdrehungen pro Minute mit einer Biofuge pico der Firma Heraeus (Osterode, Deutschland).

Die quantitative Bestimmung der extrazellulären Aminosäurekonzentrationen aus dem Kulturüberstand erfolgte mittels reversed phase HPLC (Lindroth et al., Analytical chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 µL der zu analysierenden Aminosäurelösung wurde in einer automatischen Vorsäulenderivatisierung mit 20 µL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 M; pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die Aminosäurekonzentrationen wurden über einen Vergleich mit einem externen Standard und Asparagin als zusätzlichem internen Standard berechnet.

Die Ergebnisse sind in Tabelle 2 aufgeführt.

**Tabelle 2:**

| | L-Isoleucin (mM) | |
|---|---|---|
| Stamm | 24 Stunden | 48 Stunden |
| DM368-2/pJC1 | 0,7 | 1,1 |
| DM368-2/pJC1brnEF | 1,4 | 1,8 |

Abbildungen:
   - Figur 1: Karte des das Transposon Tn5531 enthaltenden Plasmids pCGL0040. Das Transposon ist als nicht schraffierter Pfeil gekennzeichnet.

Längenangaben sind als ca.-Angaben aufzufassen. Die verwendeten Abkürzungen und Bezeichnungen haben folgende Bedeutung:
- EcoRI: Restriktionsendonuklease aus Escherichia coli
- XbaI: Restriktionsendonuklease aus Xanthomonas badrii
- ClaI: Restriktionsendonuklease aus Caryophanum latum
- SalI: Restriktionsendonuklease aus Streptomyces albus
- ScaI: Restriktionsendonuklease aus Streptomyces caespitosus
- SmaI: Restriktionsendonuklease aus Serratia marcescens
- Amp: Ampicillinresistenzgen
- Kan: Kanamycinresistenzgen
- oriBR322: Replikationsregion des Plasmides pBR322

## Patentansprüche

1. Verfahren zur Herstellung von verzweigtkettigen L-Aminosäuren durch Fermentation coryneformer Bakterien,
**dadurch gekennzeichnet,**
**dass** man coryneforme Bakterien einsetzt, in denen man das brnE- und/oder brnF-Gen überexprimiert,
wobei das brnE-Gen für ein Polypeptid kodiert, das die Funktion eines Exportproteins für eine verzweigtkettige Aminosäure besitzt, und dessen Aminosäuresequenz zu mindestens 90% identisch ist mit SEQ ID No.5, und das brnF-Gen für ein Polypeptid kodiert, das die Funktion eines Exportproteins für eine verzweigtkettige Aminosäure besitzt, und dessen Aminosäuresequenz zu mindestens 90% identisch ist mit SEQ ID No.3.

2. Verfahren gemäß Anspruch 1,
**dadurch gekennzeichnet,**
**dass** das brnE-Gen für ein Polypeptid kodiert, das die Aminosäuresequenz von SEQ ID No.5 umfasst.

3. Verfahren gemäß Anspruch 2,
**dadurch gekennzeichnet,**
**dass** das brnE-Gen die
Nukleotidsequenz von SEQ ID No.4 umfasst.

4. Verfahren gemäß Anspruch 1,
**dadurch gekennzeichnet,**
**dass** das brnF-Gen für ein Polypeptid kodiert, das die Aminosäuresequenz von SEQ ID No.3 umfasst.

5. Verfahren gemäß Anspruch 4,
**dadurch gekennzeichnet,**
**dass** das brnF-Gen die Nukleotidsequenz von SEQ ID No.2 umfasst.

6. Verfahren gemäß einem oder mehreren der Ansprüche 1 bis 5,
**dadurch gekennzeichnet,**
**dass** man das brnE-Gen und das brnF-Gen überexprimiert.

7. Verfahren gemäß Anspruch 6,
**dadurch gekennzeichnet,**
**dass** man ein Polynukleotid verwendet, das die Nukleotidsequenz von Position 101 bis 1176 von SEQ ID No.1 umfasst.

8. Verfahren gemäß den Ansprüchen 1 bis 7,
**dadurch gekennzeichnet,**
**dass** es sich bei den coryneformen Bakterien um solche der Gattung Corynebacterium handelt.

9. Verfahren gemäß Anspruch 8,
**dadurch gekennzeichnet,**
**dass** es sich bei den coryneformen Bakterien der Gattung Corynebacterium um solche der Art Corynebacterium glutamicuhandelt.

10. Verfahren gemäß einem oder mehreren der Ansprüche 1 bis 9,
**dadurch gekennzeichnet,**
**dass** man coryneformen Bakterien einsetzt, die bereits eine verzweigtkettige Aminosäure ausscheiden.

11. Verfahren gemäß einem oder mehreren der Ansprüche 1 bis 10,
**dadurch gekennzeichnet,**
**dass** man die Überexpression durch Verwendung eines starken Promotors erzielt.

12. Verfahren gemäß einem oder mehreren der Ansprüche 1 bis 10,
**dadurch gekennzeichnet,**
**dass** man die Überexpression durch Erhöhung der Kopienzahl erzielt.

13. Verfahren gemäß Anspruch 12,
**dadurch gekennzeichnet,**
**dass** man die Erhöhung der Kopienzahl unter Verwendung eines Plasmids erzielt.

14. Verfahren gemäß Anspruch 12,
**dadurch gekennzeichnet,**
**dass** man die Erhöhung der Kopienzahl durch Amplifikation im Chromosom erzielt.

15. Verfahren gemäß einem oder mehreren der Ansprüche 1-14,
**dadurch gekennzeichnet,**
**dass** man zur Herstellung von L-Isoleucin zusätzlich eines oder mehrere der Gene, ausgewählt aus der Gruppe
a) das für die Homoserin-Dehydrogenase kodierende hom-Gen oder das für eine feed back resistente Homoserin-Dehydrogenase kodierende hom^{dr}-Allel,
b) das für die Threonin-Dehydratase kodierende ilvA-Gen für eine feed back resistente Threonin- Dehydratase kodierende ilvA(Fbr)-Allel,
c) die für die Acetohydroxysäuresynthase kodierenden Gene ilvBN,
d) das für die Dihydroxysäuredehydratase kodierende ilvD-Gen,
e) das für die Pyruvat-Carboxylase kodierende pyc-Gen, und
f) das für die Malat:Chinon Oxidoreduktase kodierende mqo-Gen
überexprimiert.

16. Verfahren gemäß einem oder mehreren der Ansprüche 1-14,
**dadurch gekennzeichnet,**
**dass** man zur Herstellung von L-Leucin zusätzlich eines oder mehrere der Gene, ausgewählt aus der Gruppe
a) das für die Isopropylmalatsynthase kodierende leuA-Gen oder ein für eine feed back resistente Isopropylmalatsynthase kodierendes Allel,
b) die für die Isopropylmalatdehydratase kodierenden Gene leuC und leuD,
c) das für die Isopropylmalatdehydrogenase kodierenden leuB-Gen,
d) die für die Acetohydroxysäuresynthase kodierenden Gene ilvBN,
e) das für die Dihydroxysäuredehydratase kodierende ilvD-Gen, und
f) das für die Malat:Chinon Oxidoreduktase kodierende mqo-Gen
überexprimiert.

17. Verfahren gemäß einem oder mehreren der Ansprüche 1-14,
**dadurch gekennzeichnet,**
**dass** man zur Herstellung von L-Valin zusätzlich eines oder mehrere der Gene, ausgewählt aus der Gruppe
a) die für die Acetohydroxysäuresynthase kodierenden Gene ilvBN,
b) das für die Dihydroxysäuredehydratase kodierende ilvD-Gen, und
c) das für die Malat:Chinon Oxidoreduktase kodierende mqo-Gen
überexprimiert .

18. Verfahren gemäß mindestens einem der Ansprüche 1-17, **dadurch gekennzeichnet, dass** man folgende zusätzliche Schritte durchführt:
a) Anreicherung der gewünschten L-Aminosäure im Medium oder in den Zellen der Mikroorganismen, und
b) Isolieren der L-Aminosäure.

19. Verfahren zur Isolierung des brnE- oder brnF-Gens, **dadurch gekennzeichnet, daß** man als Indikatorstämme in diesem/diesen Gen(en) defekte Mutanten eines coryneformen Bakteriums gewinnt, die auf einem ein Isoleucin und/oder Leucin- und/oder Valin-haltiges Oligopeptid enthaltenden Nährmedium % nicht oder nur gering wachsen und
a) das brnE- oder brnF-Gen nach dem Anlegen einer Genbank identifiziert und isoliert, oder
b) im Fall der Transposon-Mutagenese auf das eine Antibiotikaresistenz enthaltende Transposon selektiert und so die gewünschten Gene erhält,
wobei das brnE-Gen für ein Polypeptid kodiert, das die Funktion eines Exportproteins für eine verzweigtkettige Aminosäure besitzt und dessen Aminosäuresequenz zu mindestens 90% identisch ist mit SEQ ID No.5 und wobei das brnF-Gen für ein Polypeptid kodiert, das die Funktion eines Exportproteins für eine verzweigtkettige Aminosäure besitzt und dessen Aminosäuresequenz zu mindestens 90% identisch ist mit SEQ ID No.3.

20. Isolierte Polynukleotide, ausgewählt aus der Gruppe
a) Polynukleotid, das für ein Polypeptid codiert, welches die Funktion eines Exportproteins für eine verzweigtkettige Aminosäure und die Aminosäuresequenz von SEQ ID No. 3 oder 5, besitzt.
b) Polynukleotid, das für ein Polypeptid codiert, welches die Funktion eines Exportproteins für eine verzweigtkettige Aminosäure und eine Aminosäuresequenz besitzt, die zu mindestens 90 % identisch ist mit der Aminosäuresequenz von SEQ ID No. 3 oder 5, und
c) Polynukleotide, das komplementär ist zu den Polynukleotiden von a) oder b).

21. Polynukleotid gemäß Anspruch 20 mit der Nukleotidsequenz SEQ ID
No. 2 .

22. Polynukleotid gemäß Anspruch 20
das für eine Aminosäuresequenz SEQ ID No. 5 kodiert.

23. Polynukleotid gemäß Anspruch 20,
mit der Nukleotidsequenz SEQ ID No. 4 .

24. Polynukleotid gemäß einem oder mehreren der Ansprüche 20 bis 23 mit der Nukleotidsequenz von SEQ ID No. 1 oder 6.

25. Vektoren, die ein Polynukleotid gemäß einem oder mehreren der Ansprüche 20 bis 24 enthalten.

26. Coryneforme Bakterien oder Escherichia coli, die
den Vektor gemäß Anspruch 25 enthalten.

27. Coryneforme Bakterien gemäß Anspruch 26, **dadurch gekennzeichnet, dass** es sich um Corynebacterium glutamicum handelt.

28. Coryneforme Bakterien gemäß einem oder mehreren der Ansprüche 26 bis 27, **dadurch gekennzeichnet, dass** es sich um einen Stamm handelt, der eine verzweigtkettige Aminosäure produziert.

29. Oligonukleotid-Primer oder Oligonukleotid-Sonde enthaltend mindestens 15 aufeinanderfolgende Nukleotide ausgewählt aus SEQ ID No.4 oder der dazu komplementären Nukleotidsequenz.

## Claims

1. Process for production of branched-chain L-amino acids by fermentation of coryneform bacteria, **characterized in that** coryneform bacteria are used in which the brnE gene and/or brnF gene are/is overexpressed, wherein the brnE gene codes for a polypeptide which has the function of an export protein for a branched-chain amino acid and whose amino acid sequence is at least 90% identical to SEQ ID No. 5, and the brnF gene codes for a polypeptide which has the function of an export protein for a branched-chain amino acid and whose amino acid sequence is at least 90% identical to SEQ ID No. 3.

2. Process according to Claim 1, **characterized in that** the brnE gene codes for a polypeptide which comprises the amino acid sequence of SEQ ID No. 5.

3. Process according to Claim 2, **characterized in that** the brnE gene comprises the nucleotide sequence of SEQ ID No. 4.

4. Process according to Claim 1, **characterized in that** the brnF gene codes for a polypeptide which comprises the amino acid sequence of SEQ ID No. 3.

5. Process according to Claim 4, **characterized in that** the brnF gene comprises the nucleotide sequence of SEQ ID No. 2.

6. Process according to one or more of Claims 1 to 5, **characterized in that** the brnE gene and the brnF gene are overexpressed.

7. Process according to Claim 6, **characterized in that** a polynucleotide is used which comprises the nucleotide sequence of position 101 to 1176 of SEQ ID No. 1.

8. Process according to Claims 1 to 7, **characterized in that** the coryneform bacteria are those of the genus Corynebacterium.

9. Process according to Claim 8, **characterized in that** the coryneform bacteria of the genus Corynebacterium are those of the species Corynebacterium glutamicum.

10. Process according to one or more of Claims 1 to 9, **characterized in that** coryneform bacteria are used which already secrete a branched-chain amino acid.

11. Process according to one or more of Claims 1 to 10, **characterized in that** overexpression is achieved by use of a strong promoter.

12. Process according to one or more of Claims 1 to 10, **characterized in that** overexpression is achieved by increase in copy number.

13. Process according to Claim 12, **characterized in that** the increase in copy number is achieved by use of a plasmid.

14. Process according to Claim 12, **characterized in that** the increase in copy number is achieved by amplification in the chromosome.

15. Process according to one or more of Claims 1 to 14, **characterized in that** L-isoleucine is produced by additionally overexpressing one or more genes selected from the group consisting of
a) the hom gene, which codes for homoserine dehydrogenase, or the hom^{dr} allele, which codes for a feedback-resistant homoserine dehydrogenase,
b) the ilvA gene, which codes for threonine dehydratase, or the ilvA(Fbr) allele which codes for a feedback-resistant threonine dehydratase,
c) the ilvBN genes, which code for acetohydroxyacid synthase,
d) the ilvD gene, which codes for dihydroxyacid dehydratase,
e) the pyc gene, which codes for pyruvate carboxylase, and
f) the mqo gene, which codes for malate:quinone oxidoreductase.

16. Process according to one or more of Claims 1 to 14, **characterized in that** L-leucine is produced by additionally overexpressing one or more genes selected from the group consisting of
a) the leuA gene, which codes for isopropylmalate synthase, or an allele which codes for a feedback-resistant isopropylmalate synthase,
b) the leuC and leuD genes, which code for isopropylmalate dehydratase,
c) the leuB gene, which codes for isopropylmalate dehydrogenase,
d) the ilvBN genes, which code for acetohydroxyacid synthase,
e) the ilvD gene, which codes for dihydroxyacid dehydratase, and
f) the mqo gene, which codes for malate:quinone oxidoreductase.

17. Process according to one or more of Claims 1 to 14, **characterized in that** L-valine is produced by additionally overexpressing one or more genes selected from the group consisting of
a) the ilvBN genes, which code for acetohydroxyacid synthase,
b) the ilvD gene, which codes for dihydroxyacid dehydratase, and
c) the mqo gene, which codes for malate:quinone oxidoreductase.

18. Process according to at least one of Claims 1 to 17, **characterized in that** the following additional steps are carried out:
a) enriching the desired L-amino acid in the medium or in the cells of the microorganisms, and
b) isolating the L-amino acid.

19. Process for isolation of the brnE gene or brnF gene, **characterized by** selecting, as indicator strains, mutants of a coryneform bacterium defective in this/these gene(s), which do not grow or grow only slightly on a culture medium containing an isoleucine- and/or leucine-and/or valine-containing oligopeptide, and
a) identifying and isolating the brnE gene or brnF gene after creating a genomic library, or
b) in the case of transposon mutagenesis, selecting for an antibiotic-resistance containing transposon and thus obtaining the desired genes,
wherein the brnE gene codes for a polypeptide which has the function of an export protein for a branched-chain amino acid and whose amino acid sequence is at least 90% identical to SEQ ID No. 5, and wherein the brnF gene codes for a polypeptide which has the function of an export protein for a branched-chain amino acid and whose amino acid sequence is a least 90% identical to SEQ ID No. 3.

20. Isolated polynucleotides selected from the group consisting of
a) polynucleotide which codes for a polypeptide which has the function of an export protein for a branched-chain amino acid and the amino acid sequence of SEQ ID No. 3 or 5,
b) polynucleotide which codes for a polypeptide which has the function of an export protein for a branched-chain amino acid and an amino acid sequence which is at least 90% identical to the amino acid sequence of SEQ ID No. 3 or 5, and
c) polynucleotide which is complementary to the polynucleotides of a) or b).

21. Polynucleotide according to Claim 20 having nucleotide sequence SEQ ID No. 2.

22. Polynucleotide according to Claim 20 coding for amino acid sequence SEQ ID No. 5.

23. Polynucleotide according to Claim 20 having nucleotide sequence SEQ ID No. 4.

24. Polynucleotide according to one or more of Claims 20 to 23 having the nucleotide sequence of SEQ ID No. 1 or 6.

25. Vectors which contain a polynucleotide according to one or more of Claims 20 to 24.

26. Coryneform bacteria or Escherichia coli which contain the vector according to Claim 25.

27. Coryneform bacteria according to Claim 26, **characterized in that** they are Corynebacterium glutamicum.

28. Coryneform bacteria according to one or more of Claims 26 to 27, **characterized in that** they are a strain which produces a branched-chain amino acid.

29. Oligonucleotide primer or oligonucleotide probe containing at least 15 sequential nucleotides selected from the group consisting of SEQ ID No. 4 or the nucleotide sequence complementary thereto.

## Revendications

1. Procédé de préparation d'acides L-aminés à chaîne ramifiée, par fermentation de bactéries corynéformes, **caractérisé en ce qu'**on utilise des bactéries corynéformes dans lesquelles on surexprime les gènes brnE et/ou brnF, le gène brnE codant pour un polypeptide qui possède la fonction d'une protéine d'export pour un acide aminé à chaîne ramifiée, et dont la séquence d'acides aminés présente une identité d'au moins 90 % avec la séquence SEQ ID N°5, et le gène brnF code pour un polypeptide qui possède la fonction d'une protéine d'export pour un acide aminé à chaîne ramifiée et dont la séquence d'acides aminés présente une identité d'au moins 90 % avec la séquence SEQ ID N°3.

2. Procédé selon la revendication 1, **caractérisé en ce que** le gène brnE code pour un polypeptide qui comprend la séquence d'acides aminés SEQ ID N°5.

3. Procédé selon la revendication 2, **caractérisé en ce que** le gène brnE comprend la séquence nucléotidique SEQ ID N°4.

4. Procédé selon la revendication 1, **caractérisé en ce que** le gène brnF code pour un polypeptide qui comprend la séquence d'acides aminés SEQ ID N°3.

5. Procédé selon la revendication 4, **caractérisé en ce que** le gène brnF comprend la séquence nucléotidique SEQ ID N°2.

6. Procédé selon l'une ou plusieurs des revendications 1 à 5, **caractérisé en ce qu'**on surexprime le gène brnE et le gène brnF.

7. Procédé selon la revendication 6, **caractérisé en ce qu'**on utilise un polynucléotide qui comprend la séquence nucléotidique allant de la position 101 à la position 1176 de SEQ ID N°1.

8. Procédé selon l'une des revendications 1 à 7, **caractérisé en ce que**, pour ce qui concerne les bactéries corynéformes, il s'agit du genre Corynebacterium.

9. Procédé selon la revendication 8, **caractérisé en ce que**, pour ce qui concerne les bactéries corynéformes du genre Corynebacterium, il s'agit de celles de l'espèce Corynebacteirum glutamicum.

10. Procédé selon l'une ou plusieurs des revendications 1 à 9, **caractérisé en ce qu'**on utilise des bactéries corynéformes qui excrètent déjà un acide aminé à chaîne ramifiée.

11. Procédé selon l'une ou plusieurs des revendications 1 à 10, **caractérisé en ce qu'**on réalise la surexpression par utilisation d'un promoteur fort.

12. Procédé selon l'une ou plusieurs des revendications 1 à 10, **caractérisé en ce qu'**on réalise la surexpression par augmentation du nombre de copies.

13. Procédé selon la revendication 12, **caractérisé en ce qu'**on réalise l'augmentation du nombre de copies par utilisation d'un plasmide.

14. Procédé selon la revendication 12, **caractérisé en ce qu'**on réalise l'augmentation du nombre de copies par une amplification dans le chromosome.

15. Procédé selon l'une ou plusieurs des revendications 1 à 14, **caractérisé en ce que**, pour préparer la L-isoleucine, on surexprime en outre un ou plusieurs des gènes choisis dans le groupe comprenant
a) le gène hom codant pour l'homosérine déshydrogénase ou l'allèle hom^{dr} codant pour une homosérine déshydrogénase résistante à la rétroaction,
b) le gène ilvA codant pour la thréonine déshydratase ou l'allèle ilvA(Fbr) codant pour une thréonine déshydratase résistante à la rétroaction,
c) les gènes ilvBN codant pour l'acétohydroxyacide synthase,
d) le gène ilvD codant pour la dihydroxyacide synthase,
e) le gène pyc codant pour la pyruvate carboxylase, et
f) le gène mqo codant pour la malate:quinone oxydoréductase.

16. Procédé selon l'une ou plusieurs des revendications 1 à 14, **caractérisé en ce que**, pour préparer la L-leucine, on surexprime en outre un ou plusieurs des gènes choisis dans le groupe comprenant
a) le gène leuA codant pour l'isopropylmalate synthase ou un allèle codant pour une isopropylmalate synthase résistante à la rétroaction,
b) les gènes leuC et leuD codant pour l'isopropylmalate déshydratase,
c) le gène leuB codant pour l'isopropylmalate déshydrogénase,
d) le gène ilvBN codant pour l'acétohydroxyacide synthase,
e) le gène ilvD codant pour la dihydroxyacide déshydratase, et
f) le gène mqo codant pour la malate:quinone oxydoréductase.

17. Procédé selon l'une ou plusieurs des revendications 1 à 14, **caractérisé en ce que**, pour préparer la L-valine, on surexprime en outre un ou plusieurs des gènes choisis dans le groupe comprenant
a) les gènes ilvBN codant pour l'acétohydroxyacide synthase,
b) le gène ilvD codant pour la dihydroxyacide déshydratase, et
c) le gène mqo codant pour la malate:quinone oxydoréductase.

18. Procédé selon au moins l'une des revendications 1 à 17, **caractérisé en ce qu'**on met en oeuvre les étapes supplémentaires suivantes :
a) enrichissement, en l'acide L-aminé souhaité, du milieu ou des cellules des microorganismes, et
b) isolement de l'acide L-aminé.

19. Procédé pour isoler le gène brnE ou brnF, **caractérisé en ce que**, en tant que souches indicatrices dans ce/ces gène(s), on obtient des mutants défectueux d'une bactérie corynéforme, qui présentent une croissance nulle, ou seulement faible, sur un milieu nutritif contenant un oligopeptide contenant de l'isoleucine et/ou de la leucine et/ou de la valine, et
a) on identifie et isole le gène brnE ou brnF après préparation d'une génothèque, ou
b) dans le cas de la mutagenèse par transposon sur le transposon contenant une résistance aux antibiotiques, on le sélectionne, et on obtient ainsi les gènes souhaités,
le gène brnE codant pour un polypeptide qui possède la fonction d'une protéine d'export pour un acide aminé à chaîne ramifiée et dont la séquence d'acides aminés présente une identité d'au moins 90 % avec la séquence SEQ ID N°5, et le gène brnF code pour un polypeptide qui possède la fonction d'une protéine d'export pour un acide aminé à chaîne ramifiée et dont la séquence d'acides aminés présente une identité d'au moins 90 % avec la SEQ ID N°3.

20. Polynucléotides isolés, choisis dans le groupe comprenant :
a) un polynucléotide codant pour un polypeptide qui possède la fonction d'une protéine d'export pour un acide aminé à chaîne ramifiée, et la séquence d'acides aminés SEQ ID N°3 ou 5,
b) un polynucléotide codant pour un polypeptide qui possède la fonction d'une protéine d'export pour un acide aminé à chaîne ramifiée et une séquence d'acides aminés qui présente une identité d'au moins 90 % avec la séquence d'acides aminés SEQ ID N°3 ou 5, et
c) un polynucléotide qui est complémentaire des polynucléotides a) ou b).

21. Polynucléotide selon la revendication 20, ayant la séquence nucléotidique SEQ ID N°2.

22. Polynucléotide selon la revendication 20, qui code pour une séquence d'acides aminés SEQ ID N°5.

23. Polynucléotide selon la revendication 20, ayant la séquence nucléotidique SEQ ID N°4.

24. Polynucléotide selon l'une ou plusieurs des revendications 20 à 23, ayant la séquence nucléotidique SEQ ID N°1 ou 6.

25. Vecteurs qui contiennent un polynucléotide selon l'une ou plusieurs des revendications 20 à 24.

26. Bactéries corynéformes ou Escherichia coli, qui contiennent le vecteur selon la revendication 25.

27. Bactéries corynéformes selon la revendication 26, **caractérisées en ce qu'**il s'agit de Corynebacterium glutamicum.

28. Bactéries corynéformes selon l'une ou plusieurs des revendications 26 à 27, **caractérisées en ce qu'**il s'agit d'une souche qui produit un acide aminé à chaîne ramifiée.

29. Amorce oligonucléotidique ou sonde oligonucléotidique contenant au moins 15 nucléotides successifs choisis dans SEQ ID N°4 ou dans la séquence nucléotidique qui lui est complémentaire.
